# EUROPÄISCHE PATENTANMELDUNG

(11) **EP 0 248 288 A2**
(43) Veröffentlichungstag der Anmeldung: **09.12.1987**
(21) Anmeldenummer: 87107440.7
(22) Anmeldetag: 22.05.1987
(51) Int. Cl.: C07D 251/34, C07D 405/06, A01N 43/64, A01N 43/72

(54) **Trisubstituierte 1,3,5-Triazin-2,4,6-trione**

(30) Priorität: 03.06.1986 DE 3618662
(71) Anmelder: BAYER AG, 51368 Leverkusen (DE)
(72) Erfinder: Führer, Wolfgang, Dr., D-5000 Köln 80 (DE); Kühle, Engelbert, Dr., D-5060 Bergisch-Gladbach 2 (DE); Adler, Alfons, Dr., D-5000 Köln 80 (DE); Hänsler, Gerd, Dr., D-5090 Leverkusen 3 (DE)

(57) **Zusammenfassung**

Neue trisubstituierte 1,3,5-Triazin-2,4,6-trione der allgemeinen Formel (I) 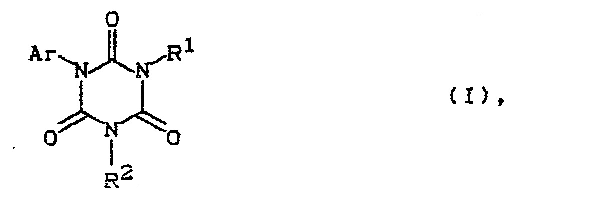 in welcher
Ar für gegebenenfalls substituiertes Aryl steht,
R¹ für einen gegebenenfalls substituierten aliphatischen oder cycloaliphatischen Rest steht und
R² für einen gegebenenfalls substituierten aliphatischen Rest steht und ihre Verwendung zur Bekämpung von Schädlingen.

Die neuen trisubstituierten 1,3,5-Triazin-2,4,6-trione der Formel (I) können hergestellt werden, wenn man geeignete 1-Aryl-1,3,5-triazin-2,4,6-trione z.B. mit geeigneten Alkylierungsmitteln umsetzt oder wenn man N.Nʹ-disubstituierte Harnstoffe mit Bischlorcarbonyl-alkylaminen umsetzt oder geeignete N,Nʹ-Dialkylharnstoffe mit Bischlorcarbonyl-arylaminen umsetzt.

## Beschreibung

Die Erfindung betrifft neue trisubstituierte 1,3,5-Triazin-2,4,6-trione, Verfahren zu ihrer Herstellung und ihre Verwendung zur Schädlingsbekämpfung, vor allem als Fungizide.

Es ist bereits bekannt geworden, daß 2-Arylamino-4,6-dichlor-s-triazine, wie z.B. das 4,6-Dichlor-N-(2-chlorphenyl)-1,3,5-triazin-2-amin, fungizide Eigenschaften besitzen (vgl. DAS 1 670 675). Die selektive fungizide Wirksamkeit dieser Stoffe ist jedoch nur auf wenige Pilze beschränkt und nicht immer ausreichend.

Es wurden neue trisubstituierte 1,3,5-Triazin-2,4,6-trione der allgemeinen Formel (I) 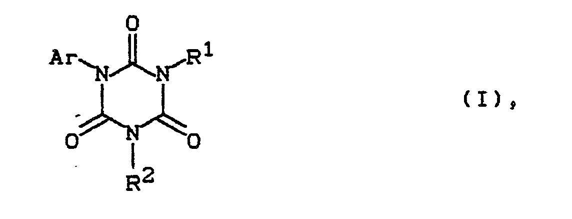 in welcher
Ar für gegebenenfalls substituiertes Aryl steht,
R¹ für einen gegebenenfalls substituierten aliphatischen oder cycloaliphatischen Rest steht und
R² für einen gegebenenfalls substituierten aliphatischen Rest steht,
gefunden.

Weiterhin wurde gefunden, daß man die neuen trisubstituierten 1,3,5-Triazin-2,4,6-trione der Formel(I) 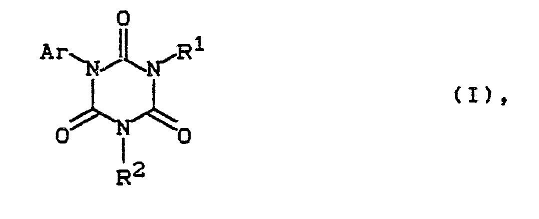 in welcher
Ar für gegebenenfalls substituiertes Aryl steht,
R¹ für einen gegebenenfalls substituierten aliphatischen oder cycloaliphatischen Rest steht und
R² für einen gegebenenfalls substituierten aliphatischen Rest steht, erhält, wenn man entweder
a) 1-Aryl-3-alkyl-1,3,5-triazin-2,4,6-trione der allgemeinen Formel (II) 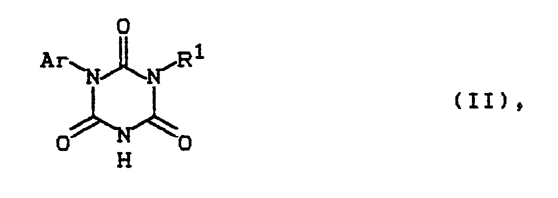 in welcher
   Ar und R¹ die oben angegebenen Bedeutungen haben,
   mit Verbindungen der allgemeinen Formel (III)
   R² - X (III),
   in welcher
   R² die oben angegebene Bedeutung hat und
   X eine Abgangsgruppe, wie z.B. Halogen oder Sulfat,
   bedeutet,
   gegebenenfalls in Gegenwart eines Verdünnungsmittels und gegebenenfalls in Gegenwart eines Säurebindemittels umsetzt oder wenn man
b) für den Fall, daß R² Alkyl bedeutet, N,Nʹ-disubstituierte Harnstoffe der allgemeinen Formel (IV)
   Ar-NH-CO-NH-R¹ (IV),
   in welcher
   Ar und R¹ die oben angegebenen Bedeutungen haben, mit einem Bischlorcarbonylamin der allgemeinen Formel 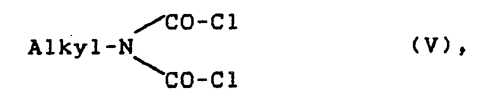 gegebenenfalls in Gegenwart eines Verdünnungsmittels und gegebenenfalls in Gegenwart eines Säurebindemittels umsetzt oder wenn man
c) für den Fall, daß R² die angegebene Bedeutung, ausgenommen Cyanalkyl hat, N,Nʹ-Dialkylharnstoffe der allgemeinen Formel (VI)
   R¹-NH-CO-NH-R²^{ʹ} (VI),
   in welcher
   R¹ die oben angegebene Bedeutung hat und
   R² die angegebene Bedeutung, ausgenommen Cyanalkyl hat,
   mit Verbindungen der allgemeinen Formel (VII) 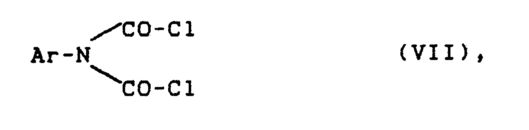 in welcher
   Ar die oben angegebene Bedeutung hat,
   umsetzt.

Schließlich wurde gefunden, daß die neuen trisubstituierten 1,3,5-Triazin-2,4,6-trione der Formel (I) sehr gute biologische Eigenschaften besitzen und vor allem zur selektiven Bekämpfung von Schadpilzen im Reis geeignet sind.

Es ist als äußerst überraschend zu bezeichnen, daß die erfindungsgemäßen Stoffe eine bessere biologische Wirksamkeit zeigen als bekannte Verbindungen aus dem Stand der Technik gleicher Wirkungsrichtung und/oder konstitutionell ähnliche Verbindungen.

Die erfindungsgemäßen trisubstituierten 1,3,5-Triazin-2,4,6-trione sind durch die Formel (I) allgemein definiert. Bevorzugt sind Verbindungen der Formel (I), in welcher
Ar für Phenyl steht, welches gegebenenfalls ein- bis fünffach, gleich oder verschieden substituiert ist durch Halogen, Alkyl mit 1 bis 4 Kohlenstoffatomen, Halogenalkyl mit 1 bis 4 Kohlenstoffatomen und 1 bis 5 gleichen oder verschiedenen Halogenatomen, Alkoxy mit 1 bis 4 Kohlenstoffatomen, Halogenalkoxy mit 1 bis 4 Kohlenstoffatomen und 1 bis 5 gleichen oder verschiedenen Halogenatomen, Alkylthio mit 1 bis 4 Kohlenstoffatomen, Halogenalkylthio mit 1 bis 4 Kohlenstoffatomen und 1 bis 5 gleichen oder verschiedenen Halogenatomen, für Halogensulfonyl, für Dialkylamino mit gleichen oder verschiedenen, geradkettigen oder verzweigten Alkylresten mit je 1 bis 4 Kohlenstoffatomen und/oder Nitro,
R¹ für geradkettiges oder verzweigtes Alkyl mit 1 bis 12 Kohlenstoffatomen steht, welches gegebenenfalls ein- oder mehrfach, gleich oder verschieden substituiert ist durch Halogen, Alkoxy mit 1 bis 3 Kohlenstoffatomen, Halogenalkoxy mit 1 oder 2 Kohlenstoffatomen und 1 bis 5 gleichen oder verschiedenen Halogenatomen, Alkylthio mit 1 bis 3 Kohlenstoffatomen, Halogenalkylthio mit 1 oder 2 Kohlenstoffatomen und 1 bis 5 gleichen oder verschiedenen Halogenatomen, Furyl und Phenyl, welches ebenfalls ein- bis fünffach, gleich oder verschieden substituiert sein kann durch Halogen; für Cycloalkyl mit 3 bis 8 Kohlenstoffatomen steht, welches gegebenenfalls ein- bis sechsfach, gleich oder verschieden substituiert ist durch Halogen, Alkyl mit 1 bis 4 Kohlenstoffatomen, Alkoxy mit 1 bis 3 Kohlenstoffatomen, Halogenalkoxy mit 1 oder 2 Kohlenstoffatomen und 1 bis 5 gleichen oder verschiedenen Halogenatomen, Alkylthio mit 1 bis 3 Kohlenstoffatomen und Halogenalkylthio mit 1 oder 2 Kohlenstoffatomen und 1 bis 5 gleichen oder verschiedenen Halogenatomen,
R² für Alkyl mit 1 bis 4 Kohlenstoffatomen, Alkenyl mit 3 bis 5 Kohlenstoffatomen, Alkinyl mit 3 bis 5 Kohlenstoffatomen, Alkoxyalkyl mit je 1 bis 3 Kohlenstoffatomen im Alkoxyteil und im Alkylteil, Alkylthioalkyl mit je 1 bis 3 Kohlenstoffatomen im Alkylthioteil und im Alkylteil, Alkoxycarbonylalkyl mit 1 bis 3 Kohlenstoffatomen im Alkoxyteil und 2 oder 3 Kohlenstoffatomen im Alkylteil oder für Cyanoalkyl mit 1 bis 5 Kohlenstoffatomen im Alkylteil steht.

Besonders bevorzugt sind Verbindungen der Formel (I), in welcher
Ar für Phenyl steht, welches gegebenenfalls ein- bis vierfach, gleich oder verschieden substituiert sein kann durch Fluor, Chlor, Brom, Alkyl mit 1 bis 3 Kohlenstoffatomen, Halogenalkyl mit 1 bis 2 Kohlenstoffatomen und 1 bis 5 Chlor- und/oder Fluoratomen, Alkoxy mit 1 bis 3 Kohlenstoffatomen, Halogenalkoxy mit 1 oder 2 Kohlenstoffatomen und 1 bis 5 Chlor- und/oder Fluoratomen, Alkylthio mit 1 oder 2 Kohlenstoffatomen, Halogenalkylthio mit 1 bis 2 Kohlenstoffatomen und 1 bis 5 Chlor- und/oder Fluoratomen, Fluorsulfonyl, Dialkylamino mit gleichen oder verschiedenen, geradkettigen oder verzweigten Alkylresten mit je 1 bis 3 Kohlenstoffatomen und/oder Nitro,
R¹ für geradkettiges oder verzweigtes Alkyl mit 1 bis 12 Kohlenstoffatomen steht, welches ein- bis dreifach gleich oder verschieden substituiert sein kann durch Fluor, Chlor, Alkoxy mit 1 oder 2 Kohlenstoffatomen, Halogenalkoxy mit 1 oder 2 Kohlenstoffatomen und 1 bis 3 Chlor- und/oder Fluoratomen, Alkylthio mit 1 oder 2 Kohlenstoffatomen, Halogenalkylthio mit 1 oder 2 Kohlenstoffatomen und 1 bis 3 Chlor- und/oder Fluoratomen, Furyl und Phenyl, welches gegebenenfalls ein- bis dreifach, gleich oder verschieden durch Chlor und Fluor substituiert sein kann, oder für Cycloalkyl mit 3 bis 6 Kohlenstoffatomen steht, welches gegebenenfalls ein- bis sechsfach, gleich oder verschieden durch Fluor, Chlor, Alkyl mit 1 oder 2 Kohlenstoffatomen, Alkoxy mit 1 oder 2 Kohlenstoffatomen, Halogenalkoxy mit 1 oder 2 Kohlenstoffatomen und 1 bis 5 Chlor- und/oder Fluoratomen, Alkylthio mit 1 oder 2 Kohlenstoffatomen, Halogenalkylthio mit 1 oder 2 Kohlenstoffatomen und mit 1 bis 3 Chlor- und/oder Fluoratomen substituiert sein kann und
R² für Alkyl mit 1 bis 3 Kohlenstoffatomen, Alkenyl mit 3 oder 4 Kohlenstoffatomen, Alkinyl mit 3 oder 4 Kohlenstoffatomen, Alkoxyalkyl mit 1 oder 2 Kohlenstoffatomen im Alkoxyteil und 1 bis 3 Kohlenstoffatomen im Alkylteil, Alkylthioalkyl mit 1 bis 2 Kohlenstoffatomen im Alkylthioteil und 1 bis 3 Kohlenstoffatomen im Alkylteil, Alkoxycarbonylalkyl mit 1 oder 2 Kohlenstoffatomen im Alkoxyteil und 2 oder 3 Kohlenstoffatomen im Alkylteil oder Cyanoalkyl mit 1 bis 3 Kohlenstoffatomen im Alkylteil steht.

Ganz besonders bevorzugt sind Verbindungen der Formel (I), in denen
Ar für gegebenenfalls ein- bis vierfach, gleich oder verschieden durch Fluor, Chlor, Methyl, Ethyl, Isopropyl, Trichlormethyl, Trifluormethyl, Tetrachlorethyl, Tetrafluorethyl, Methoxy, Ethoxy, Propoxy, Trifluormethoxy, Difluorchlormethoxy, Trifluormethylthio, Fluorsulfonyl, Dimethylamino, Diethylamino und Nitro substituiertes Phenyl steht,
R¹ für geradkettiges oder verzweigtes Alkyl mit 1 bis 12 Kohlenstoffatomen, insbesondere Isopropyl, sek.-Butyl, tert.-Butyl, sek.-Pentyl, Neopentyl, Cyclohexyl, Furylmethyl oder Benzyl steht und
R² für geradkettiges oder verzweigtes Alkyl mit 1 bis 3 Kohlenstoffatomen steht, insbesondere Methyl oder Ethyl, für Cyanmethyl, 2-Cyanethyl, Methoxycarbonylmethyl, Ethoxycarbonylmethyl, n-Propoxycarbonylmethyl, Allyl oder Propargyl steht.

Verwendet man gemäß Verfahren a) 1-(3-Trifluormethoxyphenyl)-3-(2,2-dimethylpropyl)-1,3,5-triazin-2,4,6-trion und Ethyliodid als Ausgangsstoffe, so kann der Reaktionsverlauf durch das folgende Formelschema veranschaulicht werden.

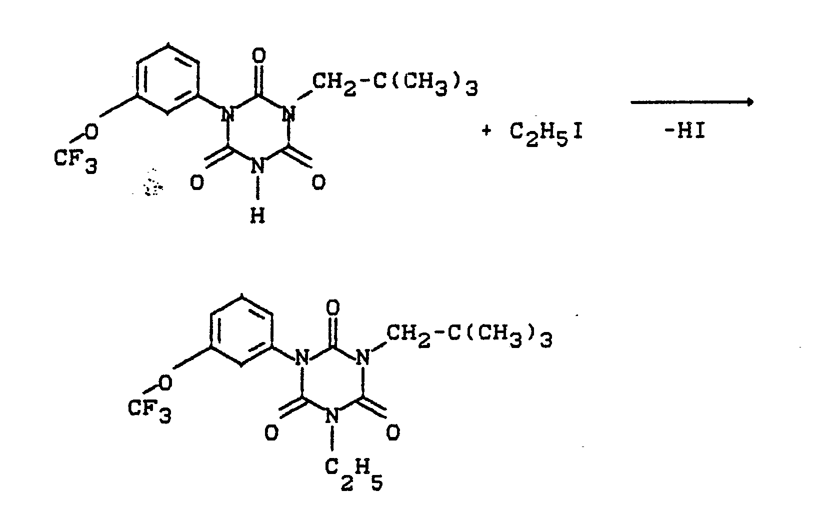

Verwendet man gemäß Verfahren b) N-(3,5-Dichlorphenyl)-Nʹ-allylharnstoff und Bis-chlorcarbonyl-N-methylamin als Ausgangsstoffe, so kann der Reaktionsverlauf durch das folgende Formelschema veranschaulicht werden:

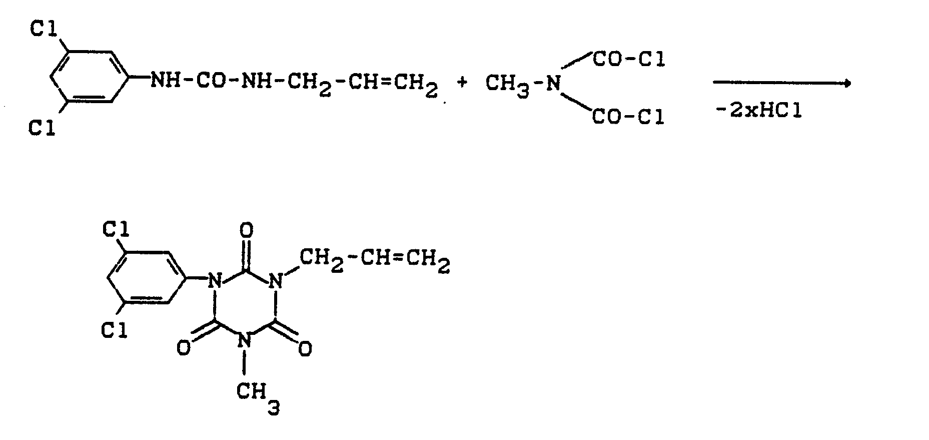

Verwendet man gemäß Verfahren c) Bis-chlorcarbonyl-N-(2,4-dichlorphenyl)-amin und N-Methyl-Nʹ-(2,2-dimethylpropyl)-harnstoff als Ausgangsstoffe, so kann der Reaktionsverlauf durch das folgende Formelschema veranschaulicht werden:

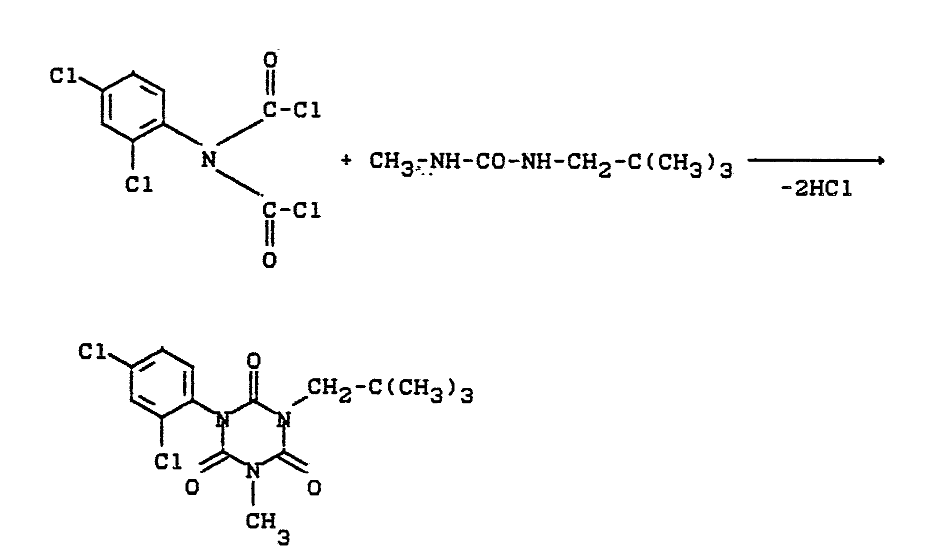

Die bei dem erfindungsgemäßen Verfahren a) als Ausgangsverbindungen benötigten disubstituierten 1,3,5-Triazin-2,4,6-trione sind durch die Formel (II) allgemein definiert. In dieser Formel haben Ar und R¹ vorzugsweise diejenigen Bedeutungen, die bereits im Zusammenhang mit der Beschreibung der erfindungsgemäßen Stoffe der Formel (I) genannt wurden.

Die 1,3,5-Triazin-2,4,6-trione der allgemeinen Formel (II) sind teilweise bekannt oder können in an sich bekannter Weise z.B. aus N,Nʹ-disubstituierten Harnstoffen (IV) und Chlorcarbonylisocyanat erhalten werden (vgl. Angew. Chem. 89 (1977), 789). Die für das Verfahren a) zu verwendenden Alkylierungsmittel der Formel (III) sind ebenfalls bekannt. Bevorzugt steht hierbei X für Chlorid, Bromid, Iodid oder Sulfat.

Die für die erfindungsgemäßen Verfahren b) oder c) zu verwendenden Harnstoffe der allgemeinen Formeln (IV) und (VI) sind an sich bekannt und durch Addition von Isocyanaten an primäre Amine zugänglich (vgl. Houben-Weyl: Methoden der organischen Chemie, Band E 4 (1983) S. 352, Thieme-Verlag, Stuttgart).

Die nach Verfahren b) oder c) zu verwendenden Bis-chlorcarbonylamine der allgemeinen Formeln (V) und (VII) sind ebenfalls bekannt (vgl. Houben-Weyl: Methoden der organischen Chemie, Band E 4 (1983), S. 1022, Thieme-Verlag, Stuttgart).

Die Reaktionstemperaturen können bei den erfindungsgemäßen Verfahren in einem größeren Temperaturbereich variiert werden. Im allgemeinen arbeitet man bei Verfahren a) zwischen 20°C und 150°C, vorzugsweise zwischen 50°C und 120°C, bei den Verfahren b) und c) im allgemeinen zwischen 0°C und 150°C, vorzugsweise zwischen 20°C und 120°C.

Bei der Durchführung der erfindungsgemäßen Verfahren setzt man die Ausgangsstoffe und gegebenenfalls die Säurebindemittel in etwa äquimolaren Mengen ein. Ein Überschuß an säurebindenden Mitteln schadet im allgemeinen nicht.

Die Umsetzungen werden bevorzugt in Gegenwart eines Verdünnungsmittels durchgeführt. Als Verdünnungsmittel kommen alle inerten organischen Lösungsmittel in Frage. Hierzu gehören vorzugsweise Kohlenwasserstoffe wie Toluol und Xylol; chlorierte Kohlenwasserstoffe wie Chlorbenzol und Chloroform; Ketone wie Aceton, Ether wie Tetrahydrofuran und Dioxan; Nitrile wie Acetonitril.

Als Säurebinder können alle üblichen säurebindenden Mittel verwendet werden. Hierzu gehören vorzugsweise tertiäre Amine wie Triethylamin und Pyridin; Alkalihydroxide wie Natrium- und Kaliumhydroxid und Alkalicarbonate und -hydrogencarbonate wie Kaliumcarbonat und Natriumhydrogencarbonat.

Die Aufarbeitung und Isolierung der erfindungsgemäßen Verbindungen erfolgt in üblicher Weise. Sie fallen im allgemeinen sofort kristallin an oder bleiben als Kristallisat nach Verdampfen des Lösungsmittels zurück.

Die erfindungsgemäßen Wirkstoffe weisen eine starke biologische Wirkung auf und können zur Bekämpfung von unerwünschten Schädlingen praktisch eingesetzt werden. Die Wirkstoffe sind für den Gebrauch z.B. als Pflanzenschutzmittel geeignet, vor allem als Fungizide.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft, aber nicht begrenzend seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea, (Konidienform: Drechslera, Syn: Helminthosporium);
Cochliobolus-Arten, wie beispielsweise Cochliobolus sativus, (Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von ober- flächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslö sungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie

Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen- Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

### Herstellungsbeispiele

Die Herstellung der erfindungsgemäßen Wirkstoffe nach Verfahren a) geht aus dem nachfolgenden Beispiel hervor.

### Beispiel 1

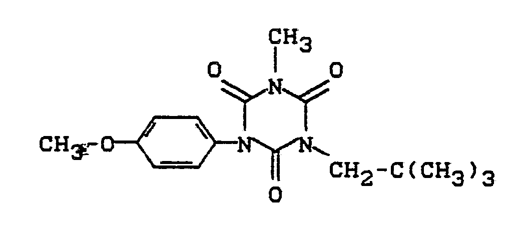

Zu einer Lösung von 10,0 g (0,033 Mol) 1-(4-Methoxyphenyl)-3-(2,2-dimethylpropyl)-1,3,5-triazin-2,4,6-trion in 100 ml absolutem Acetonitril und 9 g (0,066 Mol) Kaliumcarbonat werden bei Raumtemperatur 5,2 g (0,037 Mol) Methyliodid getropft. Das Reaktionsgemisch wird 5 Stunden unter Rückfluß gekocht. Nach dem Abkühlen werden die festen Bestandteile abgetrennt und die Mutterlauge unter vermindertem Druck eingeengt. Der nach dem Abziehen des Lösungsmittels erhaltene Rückstand wird zur Reinigung mit kaltem Wasser vermahlen. Die erhaltenen Kristalle werden abgesaugt und getrocknet. Man erhält auf diese Weise 10,0 g (95 % der Theorie) an 1-(4-Methoxyphenyl)-3-(2,2-dimethylpropyl)-5-methyl-1,3,5-triazin-2,4,6-trion in Form von Kristallen mit einem Schmelzpunkt von 134°C.

Nach der in Beispiel 1 angegebenen Methode werden auch die in der folgenden Tabelle 1 formelmäßig aufgeführten Stoffe hergestellt (identisch mit Beispiel 25).

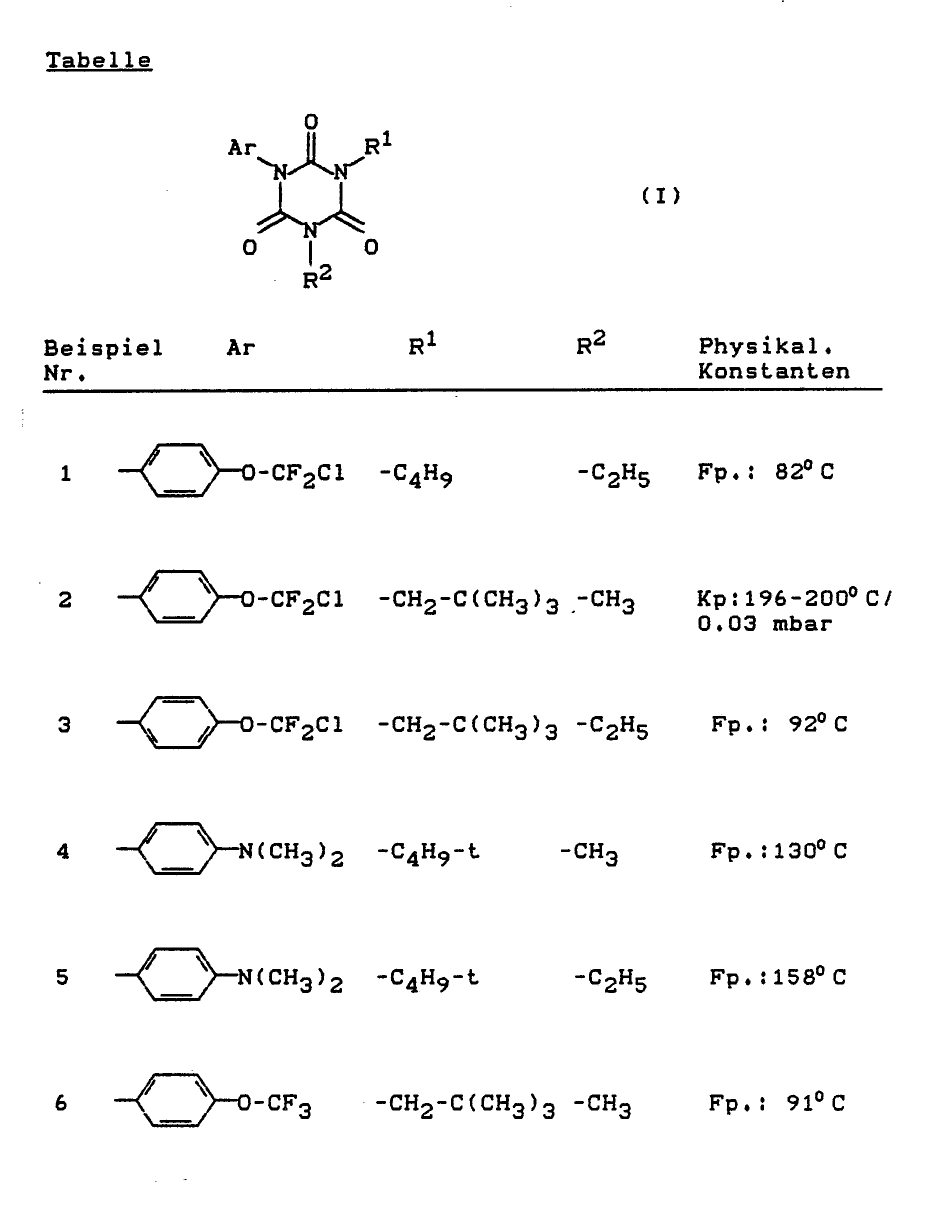

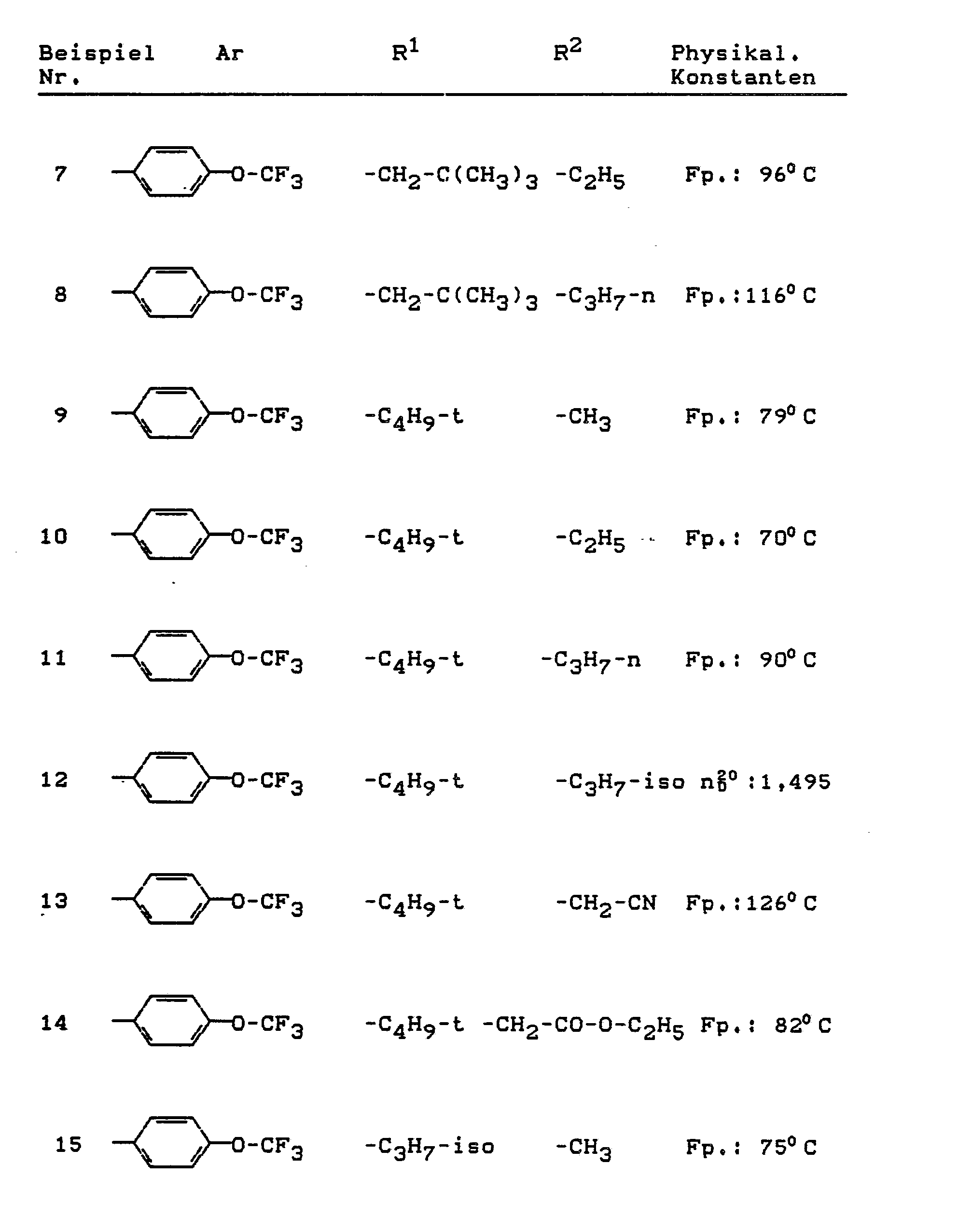

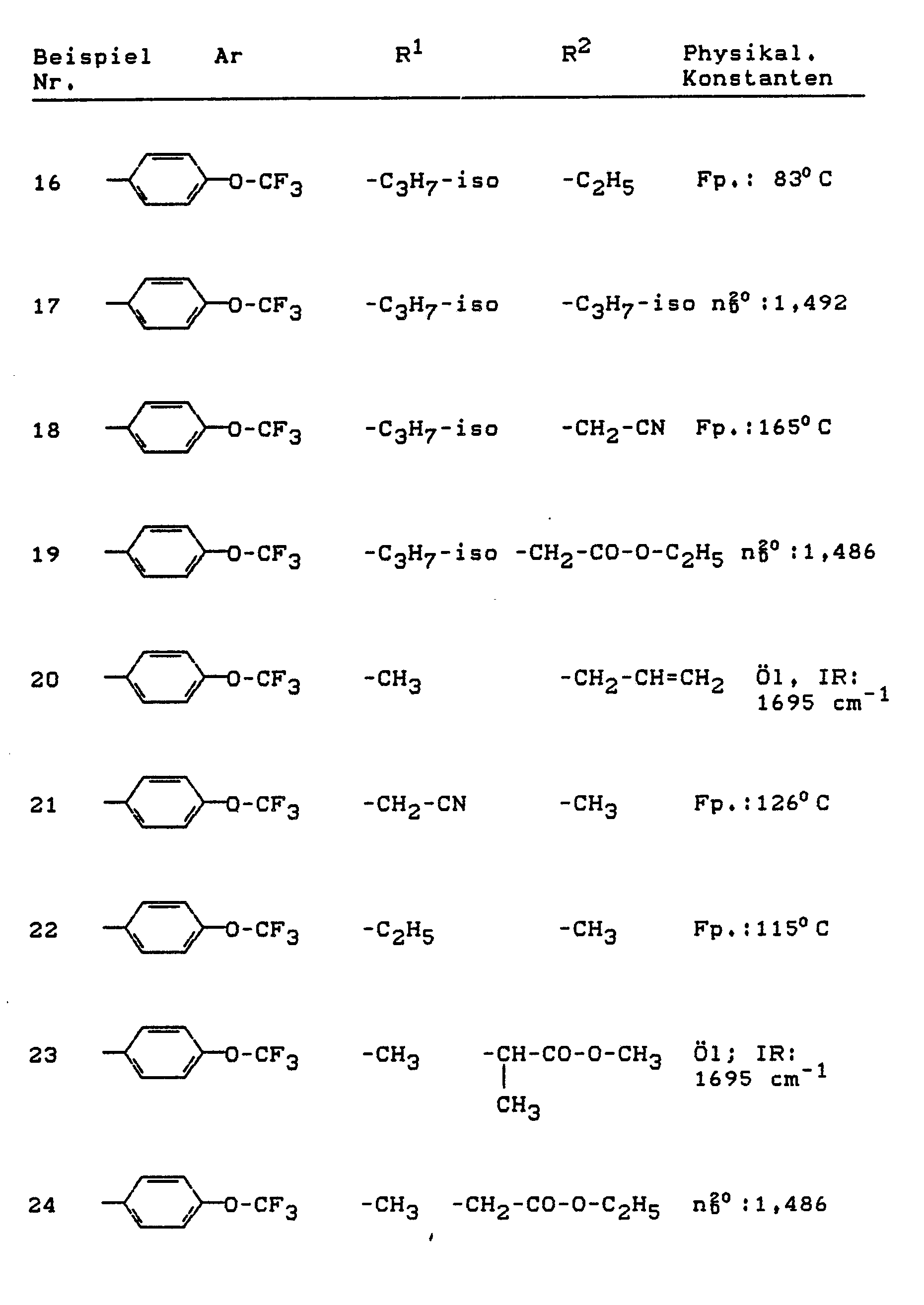

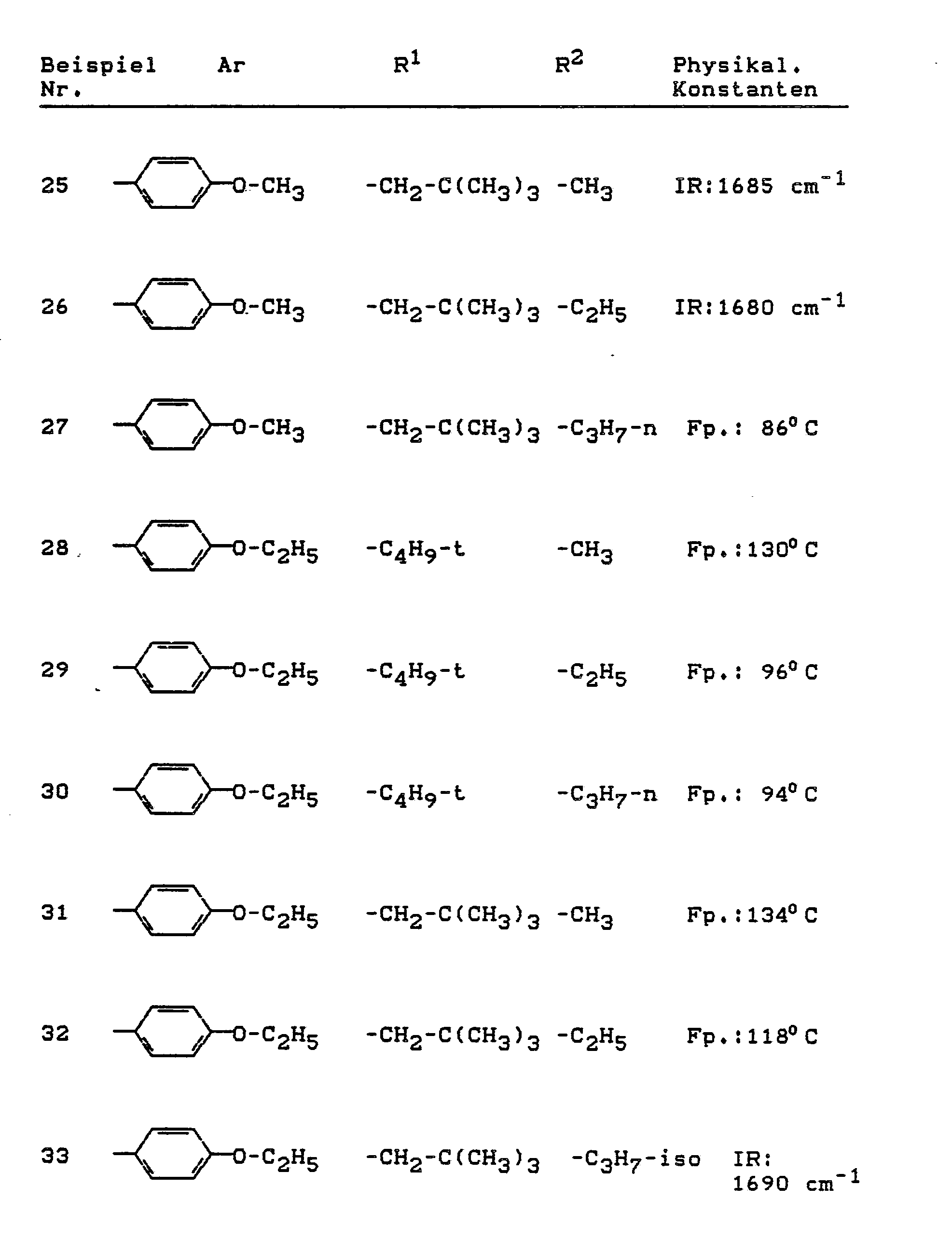

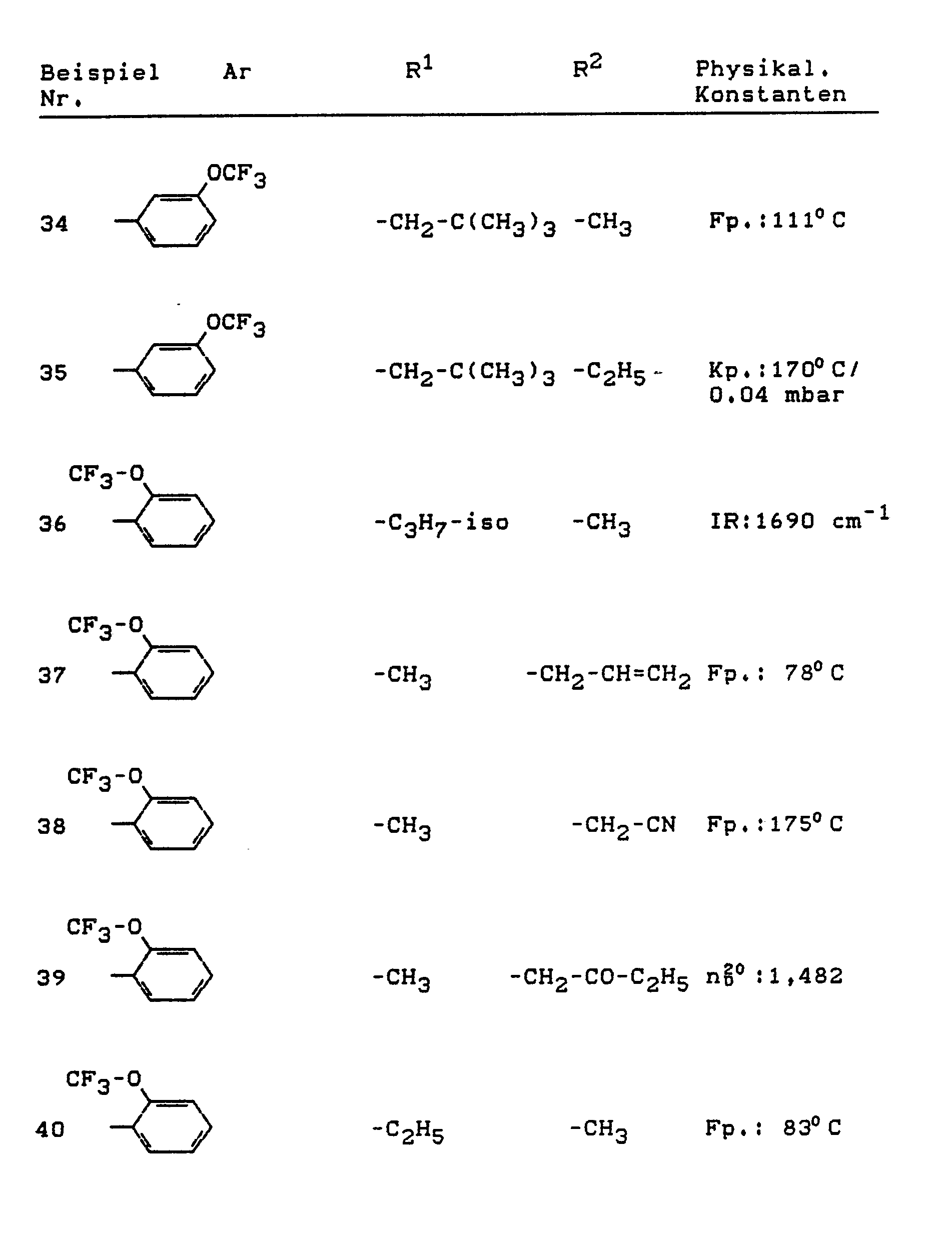

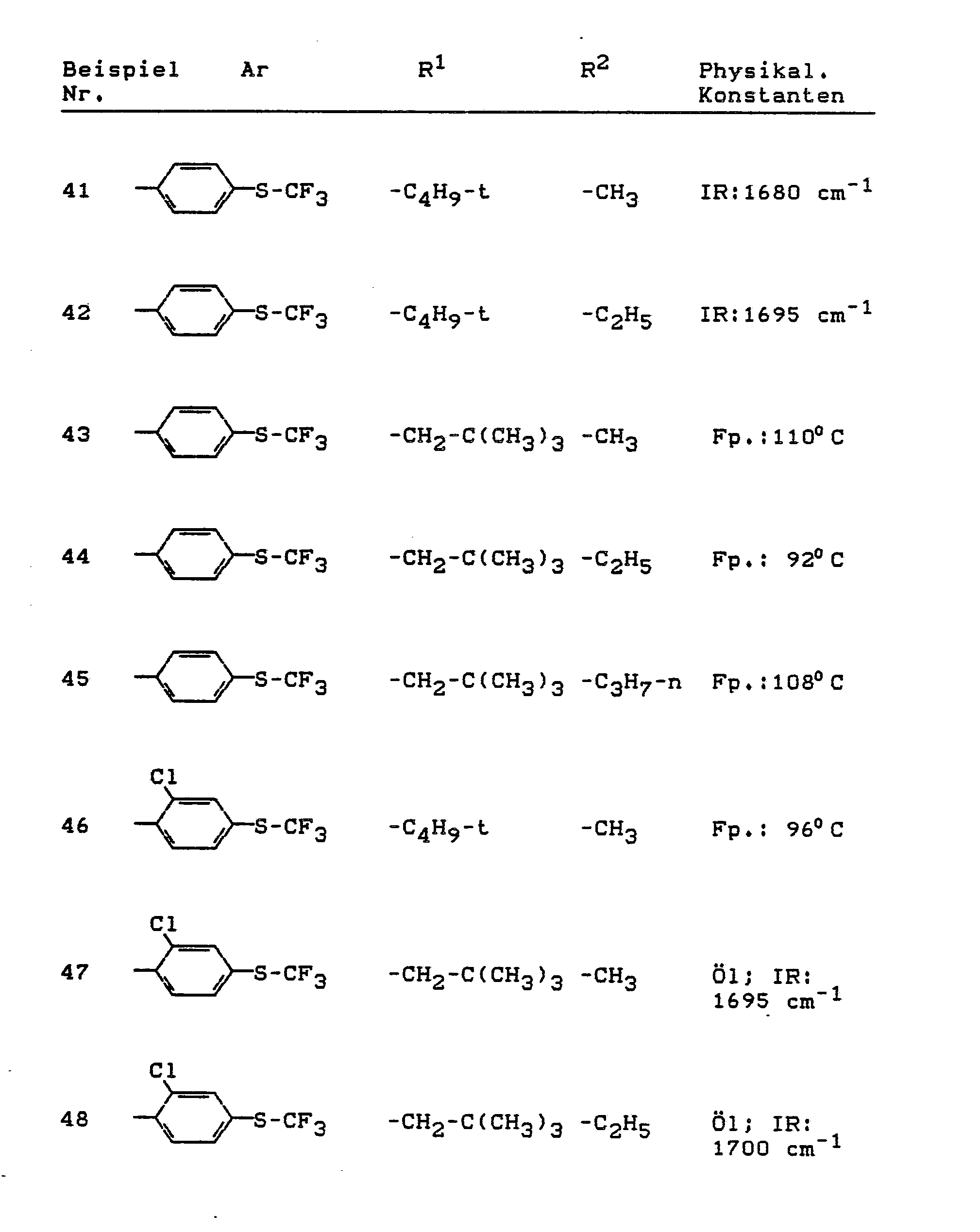

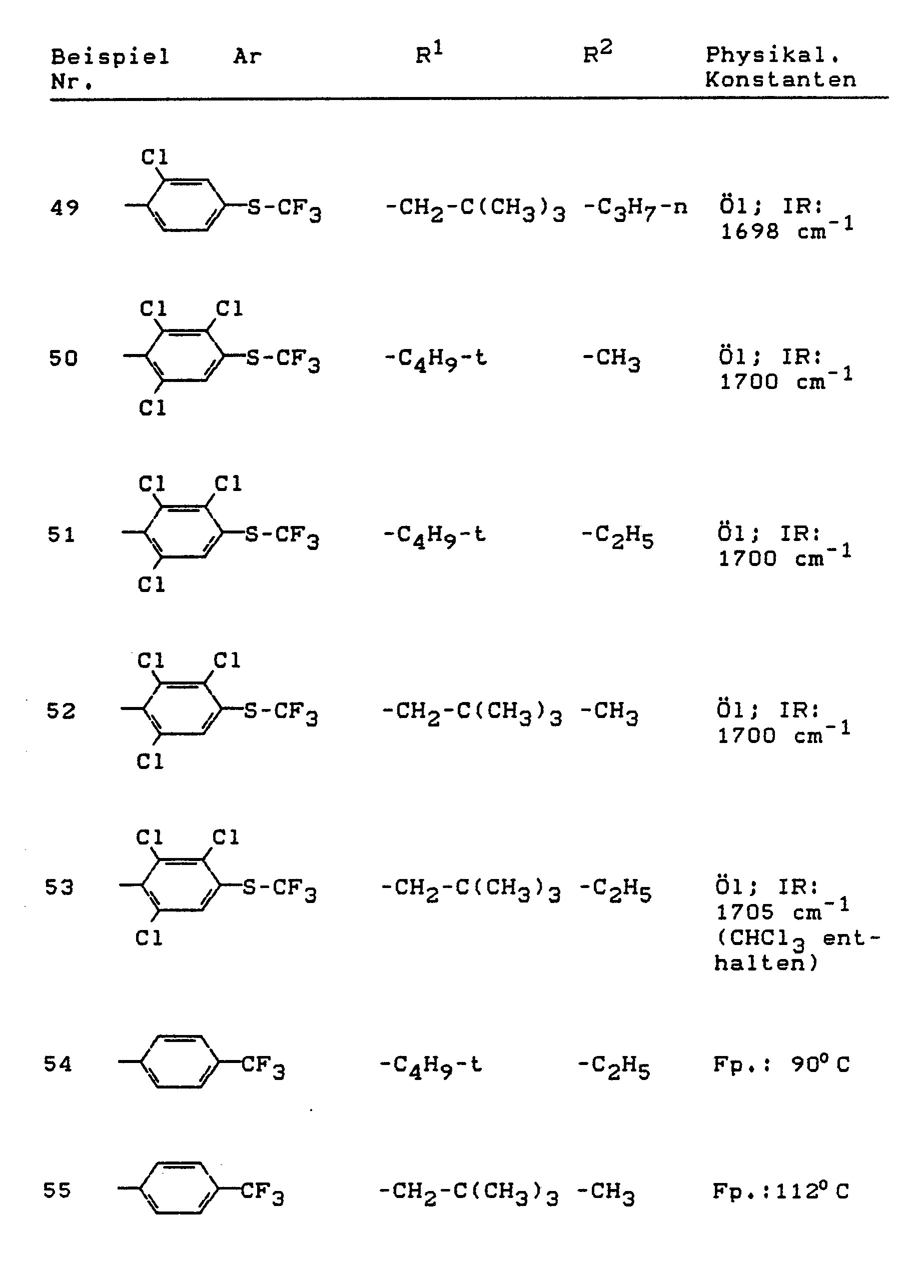

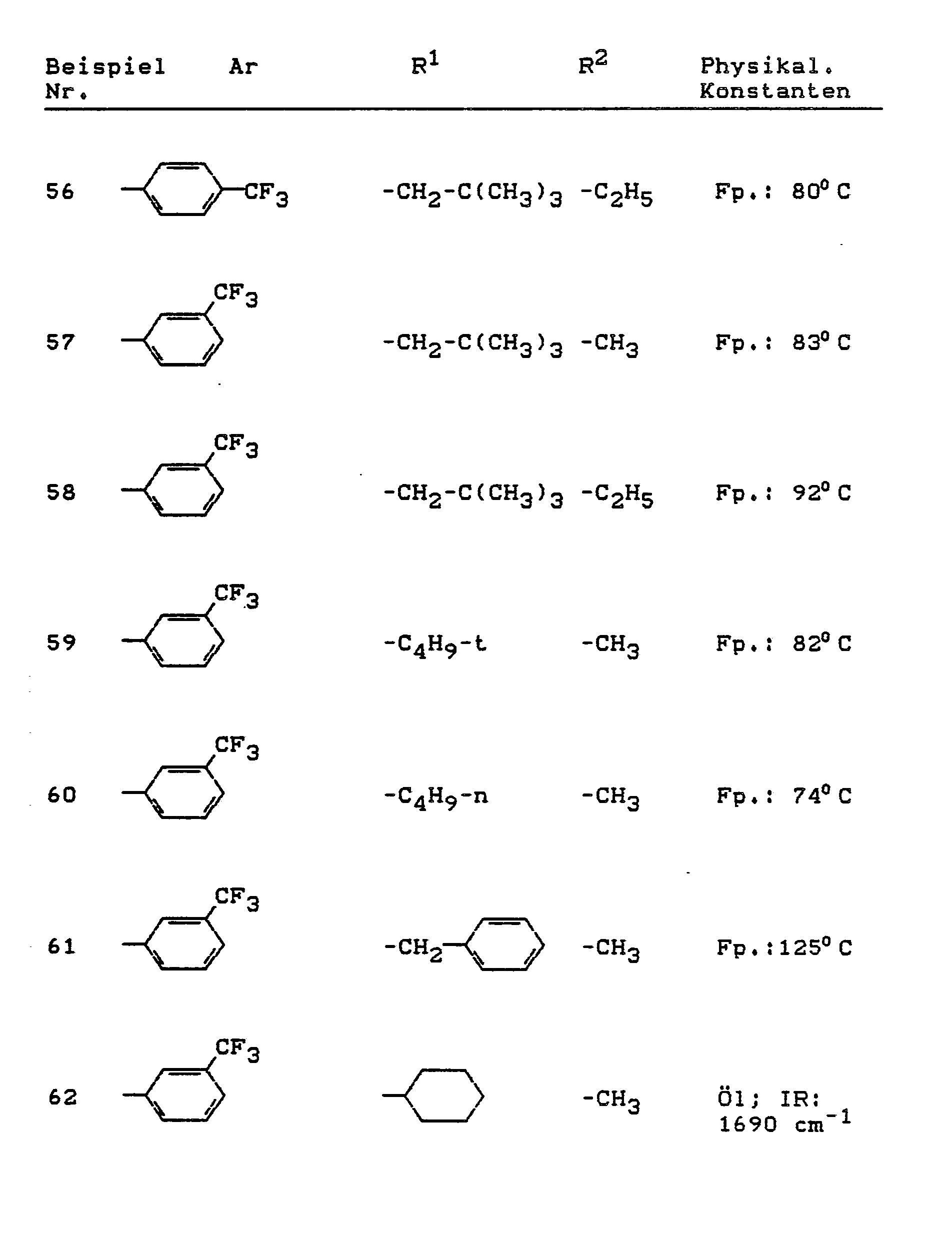

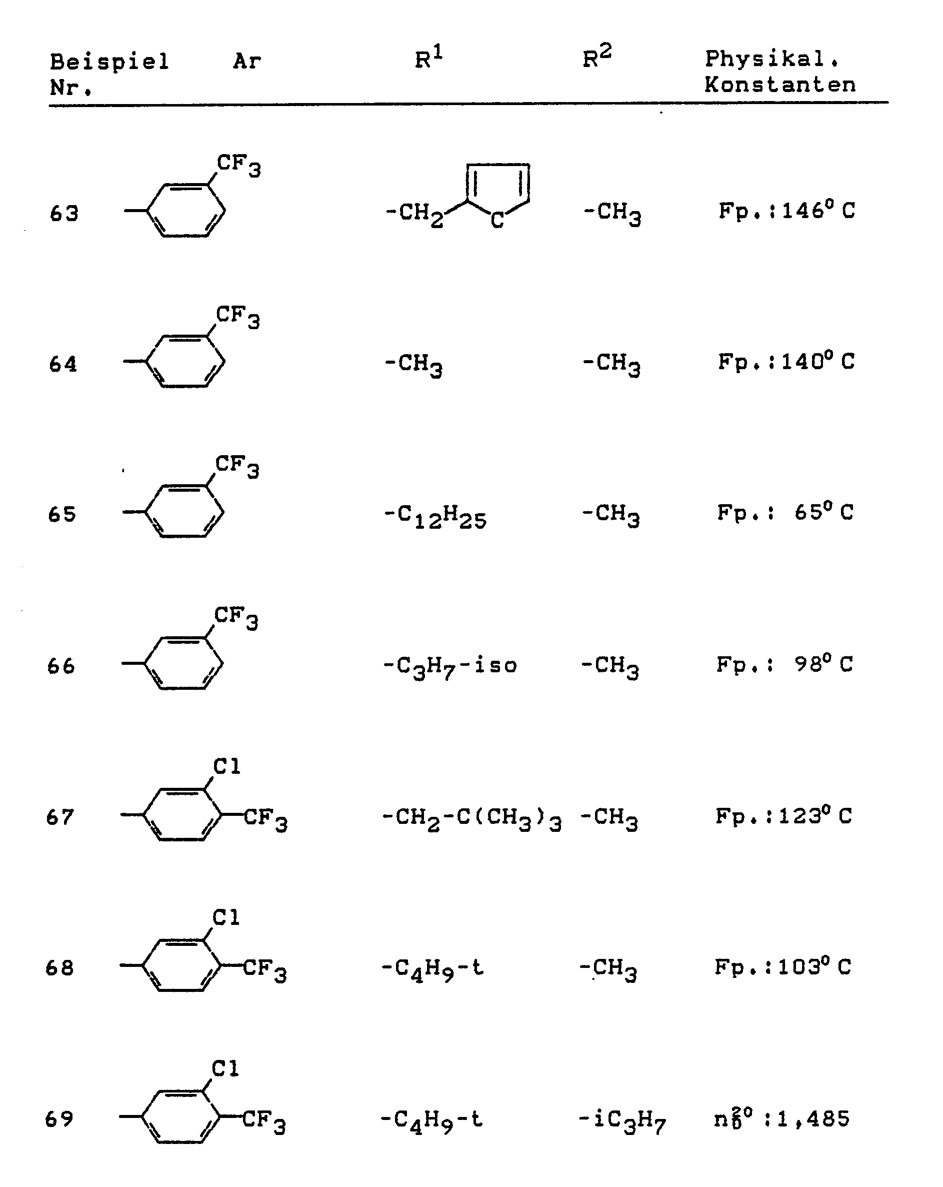

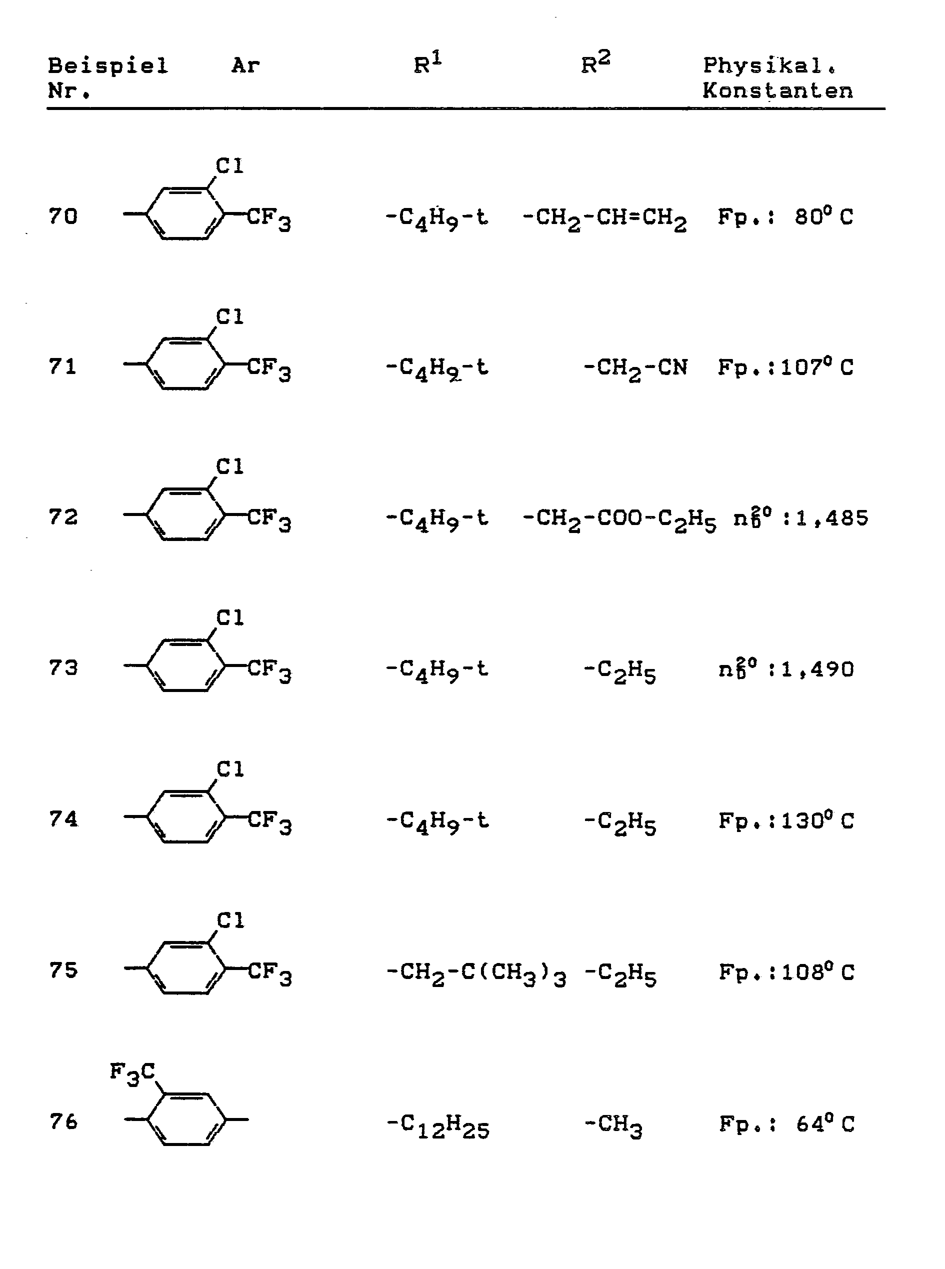

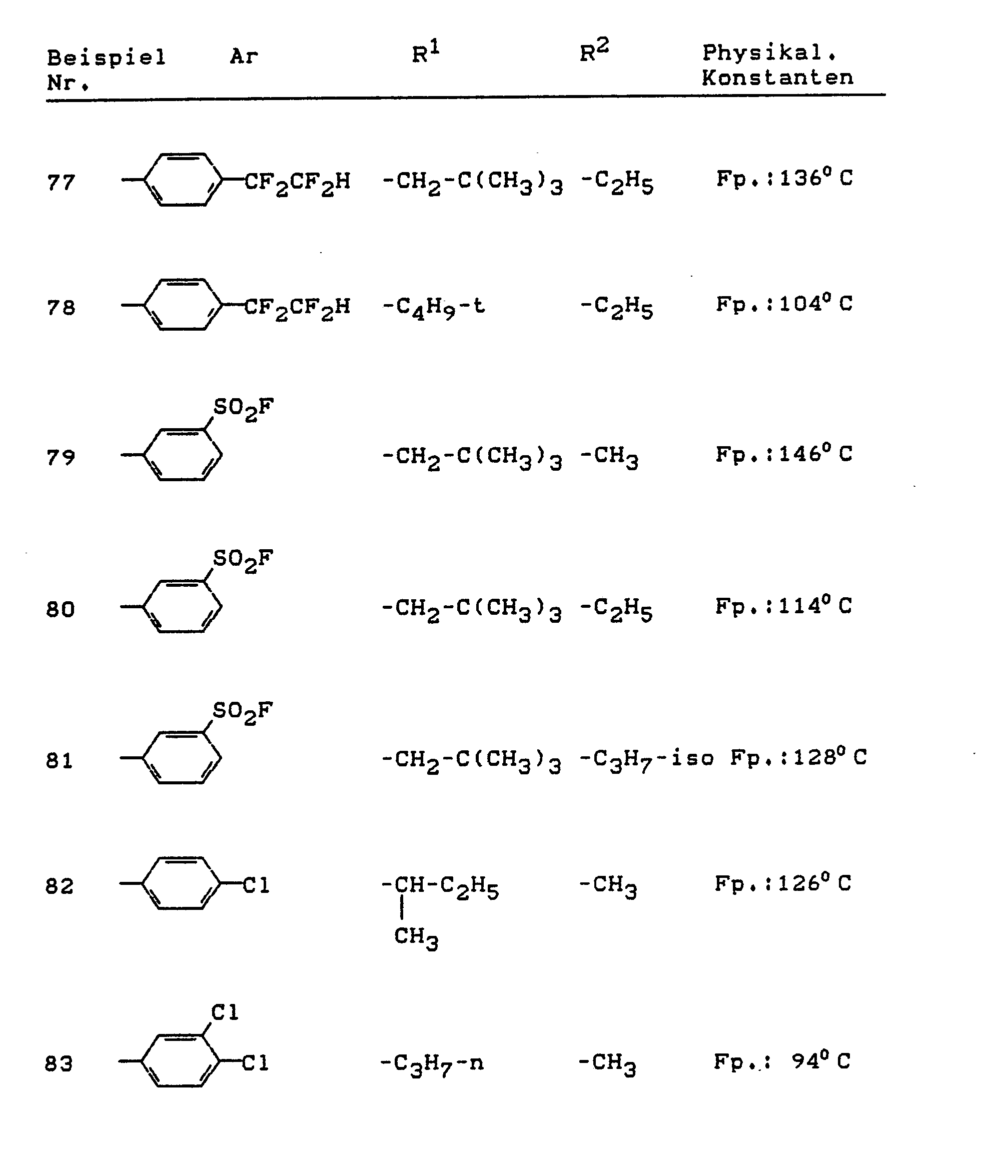

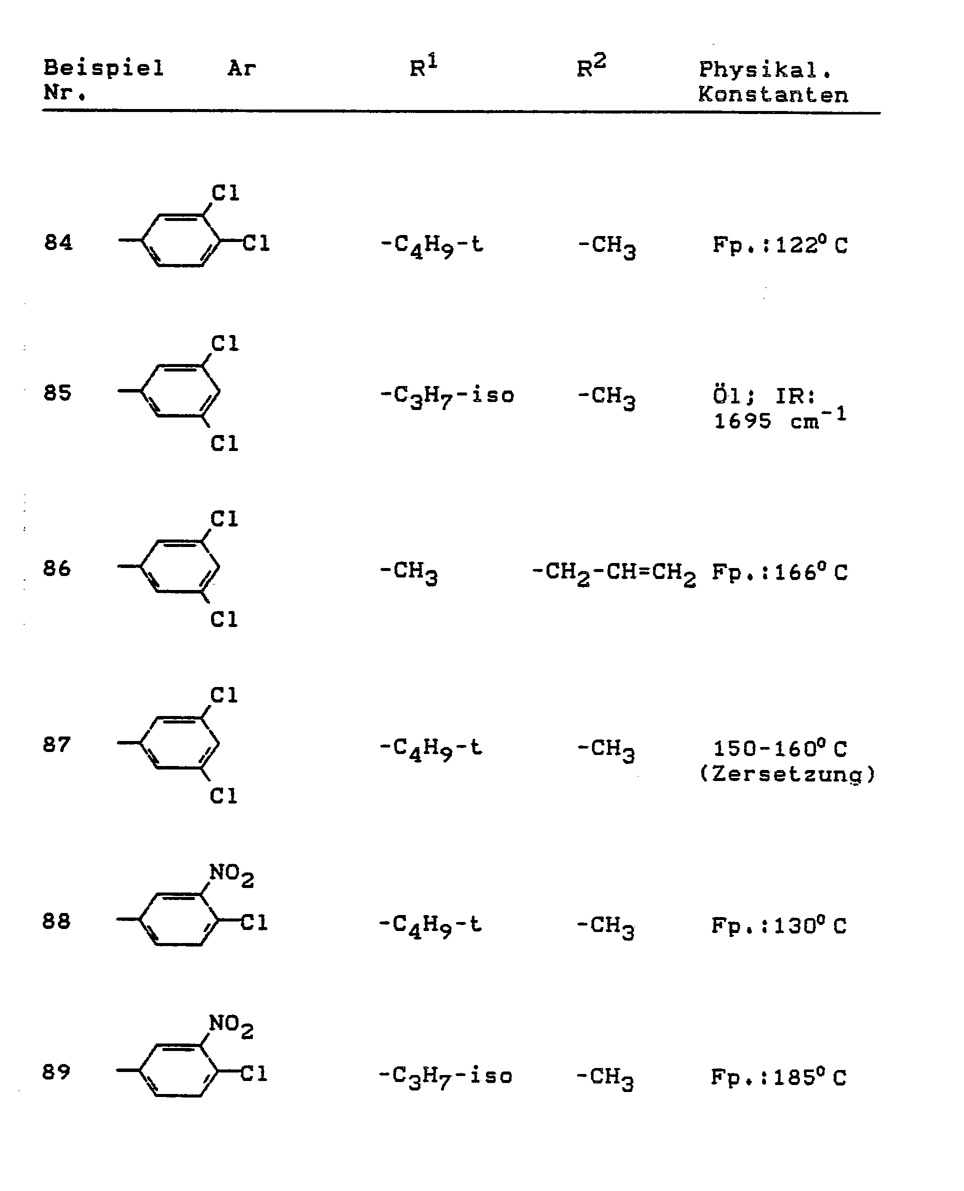

### Beispiel 90: (Verfahren b)

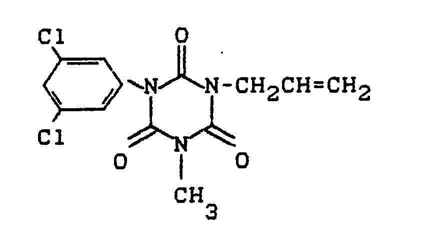

12,3 g (0,05 Mol) N(3,5-Dichlorphenyl)-Nʹ-allylharnstoff (Schmelzpunkt 133-134°C) werden in 100 ml Dioxan gelöst und mit 8 g (0,051 Mol) Bis-chlorcarbonyl-N-methylamin versetzt. Beim Erwärmen entweicht fortlaufend HCl. Bis zur Beendigung der Gasabspaltung erhitzt man 1 Stunde am Rückfluß. Man setzt zu dieser Reaktionslösung bei Raumtemperatur 150 ml Wasser zu. Hierbei kristallisiert das Reaktionsprodukt. Man saugt ab und wäscht mit Methanol nach. Ausbeute: 9 g = 55 % der Theorie; Schmelzpunkt: 165-167°C.

In analoger Weise erhält man Verbindungen der Formel

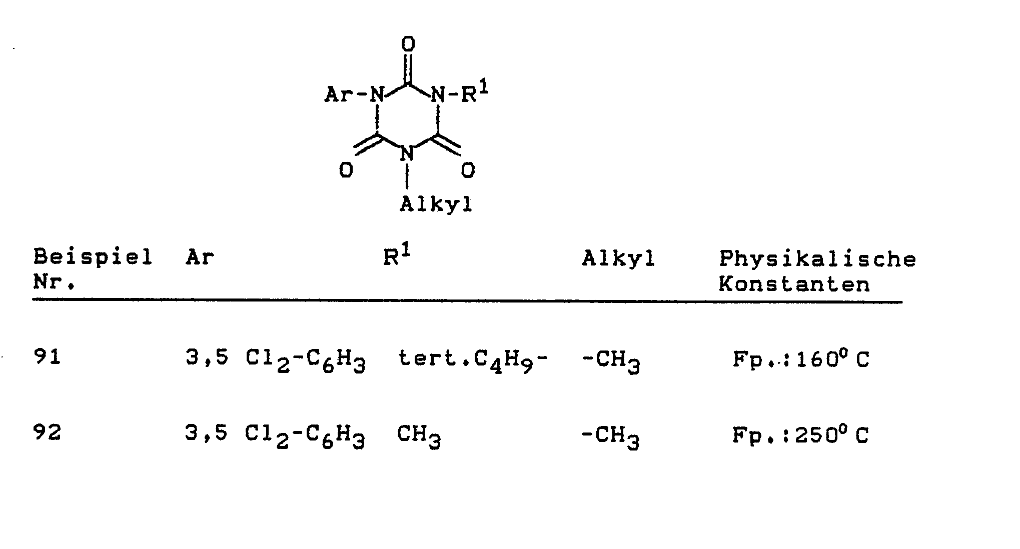

### Beispiel 93: (Verfahren C)

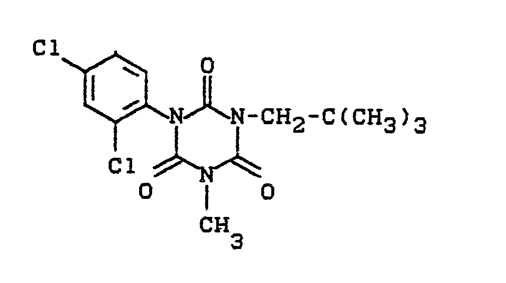

2,5 g (17,4 mMol) N-Methyl-Nʹ-(2,2-dimethylpropyl)-harnstoff werden in 20 ml Dioxan gelöst und mit 5 g (17,4 mMol) Bis-chlorcarbonyl-N-2,4-dichlorphenylamin versetzt. Beim Erwärmen entweicht fortlaufend HCl. Bis zur Beendigung der Gasabspaltung erhitzt man 1 Stunde am Rückfluß. Man setzt zu dieser Reaktionslösung 150 ml Eiswasser zu. Hierbei kristallisiert das Reaktionsprodukt. Man saugt ab und kristallisiert in einem Gemisch aus Toluol und Petrolether um.
Ausbeute: 2,2 g = 35 % der Theorie;
Schmelzpunkt = 110 - 112°C.

### Verwendungsbeispiele

### Beispiel A

### Pyricularia-Test (Reis) /protektiv

Lösungsmittel: 12,5 Gewichtsteile Aceton
Emulgator: 0,3 Gewichtsteile Alkylarylpolyglycolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß den Herstellungsbeispielen: 1, 9, 10, 14, 29, 30, 37, 54, 68, 73, 78.

### Beispiel B

### Pyricularia-Test (Reis) /systemisch

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer rel. Luftfeuchtigkeit von 100 % bis zur Auswertung.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß den Herstellungsbeispielen: 1, 9, 10, 29, 30, 54, 78.

## Patentansprüche

1. Trisubstituierte 1,3,5-Triazin-2,4,6-trione der allgemeinen Formel (I) 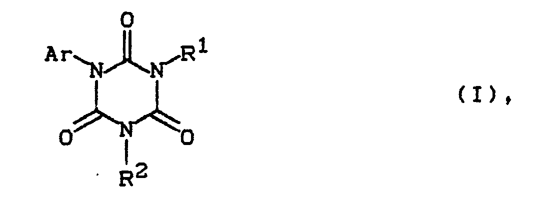 in welcher
Ar für gegebenenfalls substituiertes Aryl steht,
R¹ für einen gegebenenfalls substituierten aliphatischen oder cycloaliphatischen Rest steht und
R² für einen gegebenenfalls substituierten aliphatischen Rest steht.

2. Trisubstituierte 1,3,5-Triazin-2,4,6-trione gemäß Anspruch 1, wobei in der Formel (I)
Ar für Phenyl steht, welches gegebenenfalls einbis fünffach, gleich oder verschieden substituiert ist durch Halogen, Alkyl mit 1 bis 4 Kohlenstoffatomen, Halogenalkyl mit 1 bis 4 Kohlenstoffatomen und 1 bis 5 gleichen oder verschiedenen Halogenatomen, Alkoxy mit 1 bis 4 Kohlenstoffatomen, Halogenalkoxy mit 1 bis 4 Kohlenstoffatomen und 1 bis 5 gleichen oder verschiedenen Halogenatomen, Alkylthio mit 1 bis 4 Kohlenstoffatomen, Halogenalkylthio mit 1 bis 4 Kohlenstoffatomen und 1 bis 5 gleichen oder verschiedenen Halogenatomen, für Halogensulfonyl, für Dialkylamino mit gleichen oder verschiedenen, geradkettigen oder verzweigten Alkylresten mit je 1 bis 4 Kohlenstoffatomen und Nitro,
R¹ für geradkettiges oder verzweigtes Alkyl mit 1 bis 12 Kohlenstoffatomen steht, welches gegebenenfalls ein- oder mehrfach, gleich oder verschieden substituiert ist durch Halogen, Alkoxy mit 1 bis 3 Kohlenstoffatomen, Halogenalkoxy mit 1 oder 2 Kohlenstoffatomen und 1 bis 5 gleichen oder verschiedenen Halogenatomen, Alkylthio mit 1 bis 3 Kohlenstoffatomen, Halogenalkylthio mit 1 oder 2 Kohlenstoffatomen und 1 bis 5 gleichen oder verschiedenen Halogenatomen, Furyl und Phenyl, welches ebenfalls ein- bis fünffach, gleich oder verschieden substituiert sein kann durch Halogen; für Cycloalkyl mit 3 bis 8 Kohlenstoffatomen steht, welches gegebenenfalls ein- bis sechsfach, gleich oder verschieden substituiert ist durch Halogen, Alkyl mit 1 bis 4 Kohlenstoffatomen, Alkoxy mit 1 bis 3 Kohlenstoffatomen Halogenalkoxy mit 1 oder 2 Kohlenstoffatomen und 1 bis 5 gleichen oder verschiedenen Halogenatomen, Alkylthio mit 1 bis 3 Kohlenstoffatomen und Halogenalkylthio mit 1 oder 2 Kohlenstoffatomen und 1 bis 5 gleichen oder verschiedenen Halogenatomen und
R² für Alkyl mit 1 bis 4 Kohlenstoffatomen, Alkenyl mit 3 bis 5 Kohlenstoffatomen, Alkinyl mit 3 bis 5 Kohlenstoffatomen, Alkoxyalkyl mit je 1 bis 3 Kohlenstoffatomen im Alkoxyteil und im Alkylteil, Alkylthioalkyl mit je 1 bis 3 Kohlenstoffatomen im Alkylthioteil und im Alkylteil, Alkoxycarbonylalkyl mit 1 bis 3 Kohlenstoffatomen im Alkoxyteil und 2 oder 3 Kohlenstoffatomen im Alkylteil oder für Cyanoalkyl mit 1 bis 5 Kohlenstoffatomen im Alkylteil steht.

3. Trisubstituierte 1,3,5-Triazin-2,4,6-trione gemäß Anspruch 1, wobei in der Formel (I)
Ar für Phenyl steht, welches gegebenenfalls ein- bis vierfach, gleich oder verschieden substituiert sein kann durch Fluor, Chlor, Brom, Alkyl mit 1 bis 3 Kohlenstoffatomen, Halogenalkyl mit 1 bis 3 Kohlenstoffatomen und 1 bis 5 Chlor- und/oder Fluoratomen, Alkoxy mit 1 bis 3 Kohlenstoffatomen, Halogenalkoxy mit 1 oder 2 Kohlenstoffatomen und 1 bis 5 Chlor-und/oder Fluoratomen, Alkylthio mit 1 oder 2 Kohlenstoffatomen, Halogenalkylthio mit 1 bis 3 Kohlenstoffatomen und 1 bis 5 Chlor- und/oder Fluoratomen, Fluorsulfonyl, Dialkylamino mit gleichen oder verschiedenen, geradkettigen oder verzweigten Alkylresten mit je 1 bis 3 Kohlenstoffatomen und Nitro,
R¹ für geradkettiges oder verzweigtes Alkyl mit 1 bis 12 Kohlenstoffatomen steht, welches ein- bis dreifach, gleich oder verschieden substituiert sein kann durch Fluor, Chlor, Alkoxy mit 1 oder 2 Kohlenstoffatomen, Halogenalkoxy mit 1 oder 2 Kohlenstoffatomen und 1 bis 3 Chlor- und/oder Fluoratomen, Alkylthio mit 1 oder 2 Kohlenstoffatomen, Halogenalkylthio mit 1 oder 2 Kohlenstoffatomen und 1 bis 3 Chlor- und/oder Fluoratomen, Furyl und Phenyl, welches gegebenenfalls ein- bis dreifach, gleich oder verschieden durch Chlor und Fluor substituiert sein kann, oder für Cycloalkyl mit 3 bis 6 Kohlenstoffatomen steht, welches gegebenenfalls ein- bis sechsfach, gleich oder verschieden durch Fluor, Chlor, Alkyl mit 1 oder 2 Kohlenstoffatomen, Alkoxy mit 1 oder 2 Kohlenstoffatomen, Halogenalkoxy mit 1 oder 2 Kohlenstoffatomen und 1 bis 5 Chlor- und/oder Fluoratomen, Alkylthio mit 1 oder 2 Kohlenstoffatomen, Halogenalkylthio mit 1 oder 2 Kohlenstoffatomen und mit 1 bis 3 Chlor- und/oder Fluoratomen steht und
R² für Alkyl mit 1 bis 3 Kohlenstoffatomen, Alkenyl mit 3 oder 4 Kohlenstoffatomen, Alkinyl mit 3 oder 4 Kohlenstoffatomen, Alkoxyalkyl mit 1 oder 2 Kohlenstoffatomen im Alkoxyteil und 1 bis 3 Kohlenstoffatomen im Alkylteil, Alkylthioalkyl mit 1 bis 2 Kohlenstoffatomen im Alylthioteil und 1 bis 3 Kohlenstoffatomen im Alkylteil, Alkoxycarbonylalkyl mit 1 oder 2 Kohlenstoffatomen im Alkoxyteil und 2 oder 3 Kohlenstoffatomen im Alkylteil oder Cyanoalkyl mit 1 bis 3 Kohlenstoffatomen im Alkylteil steht.

4. Trisubstituierte 1,3,5-Triazin-2,4,6-trione gemäß Anspruch 1, wobei in der Formel (I)
Ar für gegebenenfalls ein- bis vierfach, gleich oder verschieden durch Fluor, Chlor, Methyl, Ethyl, iso-Propyl, Trichlormethyl, Trifluormethyl, Tetrachlorethyl, Tetrafluorethyl, Methoxy, Ethoxy, Propoxy, Trifluormethoxy, Difluorchlormethoxy, Trifluormethylthio, Fluorsulfonyl, Chlorsulfonyl, Dimethylamino, Diethylamino und/oder Nitro substituiertes Phenyl steht,
R¹ für geradkettiges oder verzweigtes Alkyl mit 1 bis 12 Kohlenstoffatomen, für Cyclohexyl, Furylmethyl oder Benzyl steht und
R² für geradkettiges oder verzweigtes Alkyl mit 1 bis 5 Kohlenstoffatomen steht, für Cyanmethyl, 2-Cyanethyl, Methoxycarbonylmethyl, Ethoxycarbonylmethyl, n-Propoxycarbonylmethyl, Allyl oder Propargyl steht.

5. Verfahren zur Herstellung von trisubstituierten 1,3,5-Triazin-2,4,6-trionen der allgemeinen Formel (I) 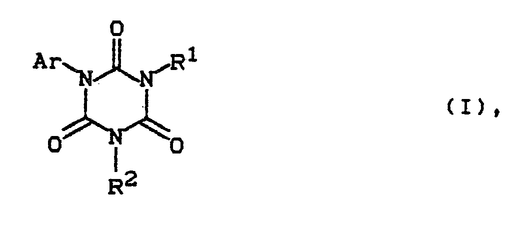 in welcher
Ar für gegebenenfalls substituiertes Aryl steht,
R¹ für einen gegebenenfalls substituierten aliphatischen oder cycloaliphatischen Rest steht und
R² für einen gegebenenfalls substituierten aliphatischen Rest steht,
dadurch gekennzeichnet, daß man
a) 1-Aryl-1,3,5-triazin-2,4,6-trione der allgemeinen Formel (II) 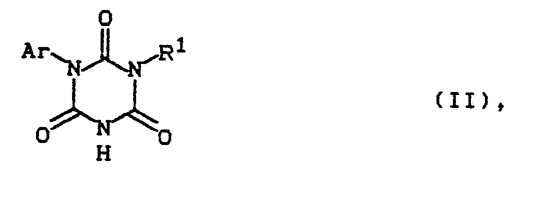 in welcher
Ar und R¹ die oben angegebenen Bedeutungen haben,
mit Verbindungen der allgemeinen Formel (III)
R²-X (III),
in welcher
R² die oben angegebene Bedeutung hat und
X eine Abgangsgruppe, wie z.B. Halogen oder Sulfat,
bedeutet,
gegebenenfalls in Gegenwart eines Verdünnungsmittels und gegebenenfalls in Gegenwart eines Säurebindemittels umsetzt oder
b) für den Fall, daß R² Alkyl bedeutet, einen N,Nʹ-disubstituierten Harnstoff der allgemeinen Formel (IV)
Ar-NH-CO-NH-R¹ (IV),
in welcher
Ar und R¹ die oben angegebenen Bedeutungen haben, mit einem Bischlorcarbonylamin der allgemeinen Formel (V) 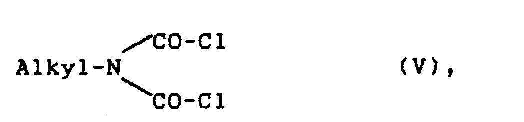 gegebenenfalls in Gegenwart eines Verdünnungsmittels und gegebenenfalls in Gegenwart eines Säurebindemittels umsetzt oder
c) für den Fall, daß R² die angegebene Bedeutung, ausgenommen Cyanalkyl hat, einen N,Nʹ-Dialkylharnstoff der allgemeinen Formel (VI)
R¹-NH-CO-NH-R²^{ʹ} (VI),
in welcher
R¹ die oben angegebene Bedeutung hat und
R² die angegebene Bedeutung, ausgenommen Cyanalkyl hat,
mit einer Verbindung der allgemeinen Formel (VII) 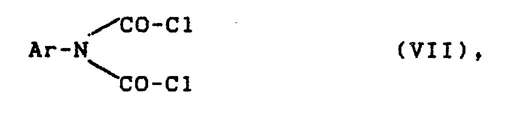 in welcher
Ar die oben angegebene Bedeutung hat, umsetzt.

6. Schädlingsbekämpfungsmittel, gekennzeichnet durch einen Gehalt an mindestens einem trisubstituierten 1,3,5-Triazin-2,4,6-trion der Formel (I) gemäß den Ansprüchen 1 bis 5.

7. Verwendung von trisubstituierten 1,3,5-Triazin-2,4,6-trionen der Formel (I) nach den Ansprüchen 1 bis 5 zur Bekämpfung von Schädlingen.

8. Verfahren zur Bekämpfung von Schädlingen, dadurch gekennzeichnet, daß man trisubstituierte 1,3,5-Triazin-2,4,6-trione der Formel (I) nach den Ansprüchen 1 bis 5 auf Schädlinge und/oder ihren Lebensraum einwirken läßt.

9. Verfahren zur Herstellung von Schädlingsbekämpfungsmitteln, dadurch gekennzeichnet, daß man trisubstituierte 1,3,5-Triazin-2,4,6-trione der Formel (I) nach den Ansprüchen 1 bis 5 mit Streckmitteln und/oder oberflächenaktiven Mitteln vermischt.
